# EUROPÄISCHE PATENTSCHRIFT

(11) **EP 1 438 009 B1**
(45) Veröffentlichungstag und Bekanntmachung des Hinweises auf die Patenterteilung: **23.01.2008**
(21) Anmeldenummer: 02800583.3
(22) Anmeldetag: 30.09.2002
(51) Int. Cl.: A61Q 15/00, A61K 8/86, A61K 8/34, A61K 8/26, A61K 8/04, A61K 8/37, A61K 8/06, B05B 17/00

(54) **ANTITRANSPIRANTPRODUKT AUF BASIS VON MIKROEMULSIONEN**
ANTIPERSPIRANT PRODUCT BASED ON MICROEMULSIONS
PRODUIT ANTITRANSPIRANT A BASE DE MICROEMULSIONS

(30) Priorität: 06.10.2001 DE 10149362
(43) Veröffentlichungstag der Anmeldung: 21.07.2004
(73) Patentinhaber: Beiersdorf AG, 20245 Hamburg (DE)
(72) Erfinder: KUX, Ulrich, 22559 Hamburg (DE); CIERPISZ, Yvonne, 20251 Hamburg (DE); MÄHLMANN, Kurt, 21629 Neu Wulmstorf (DE); MENZEL, Norbert, 21244 Buchholz (DE); DIEC, Khiet, Hien, 22523 Hamburg (DE)
(86) Internationale Anmeldenummer: PCT/EP2002/010932
(87) Internationale Veröffentlichungsnummer: WO 2003/030852

(56) Entgegenhaltungen:
- EP-A- 0 000 313
- EP-A- 0 343 843
- EP-A- 0 558 186
- DE-A- 19 509 079
- US-A- 5 980 874

## Beschreibung

Die vorliegende Erfindung betrifft ein kosmetisches Deoprodukt, welches eine Kombination aus Pack- und Applikationsmittel und einer dünnflüssigen Öl-in-Wasser-Mikroemulsion mit einem hohen Gehalt an Antitranspirantsalz ist und mit Hilfe einer Zerstäuberpumpe gleichmäßig appliziert werden kann.

Die menschliche Haut ist mit zwei bis drei Millionen Schweißdrüsen durchsetzt. Diese sind Tag und Nacht im Einsatz, um Feuchtigkeit an die Hautoberfläche zu befördern und so der Überhitzung des Organismus vorzubeugen. Der austretende Schweiß sorgt bei seiner Verdunstung für die notwendige Kühlung.
Täglich werden im Normalfall 0,5 bis 1 Liter Schweiß produziert. Bei Belastung des Körpers und erhöhtem Stoffwechsel kann es ein Vielfaches an produziertem Schweiß sein. Im Laufe der Entwicklung eines Menschen kommt es zur Ausbildung zweier Arten von Schweißdrüsen. Von Geburt an besitzt der Mensch nur ekkrine Schweißdrüsen (kleine Schweißdrüsen), mit Beginn der Pubertät jedoch kommt es hauptsächlich im Bereich der Achseln und im Anal- und Genitalbereich zur Ausbildung der apokrinen Schweißdrüsen (große Schweißdrüsen). Erst diese sorgen in Zusammenhang mit Hautbakterien, die den geruchlosen Schweiß zersetzen, zu den bekannten unangenehmen Gerüchen. Der Schweißgeruch ist personenspezifisch und bei jedem Menschen unterschiedlich ausgeprägt. Durch einfaches Waschen lässt sich bei den meisten Menschen nur eine kurzfristige Besserung erzielen, so dass es oft genug nicht ohne die Anwendung von Deowirkstoffen geht.
Um eine Deowirkung zu erreichen gibt es verschiedene Wege, die im Normalfall kombiniert angewendet werden.

Der Einsatz von Antitranspirantien, die die Schweißproduktion durch Blockierung der Schweißdrüsenausgänge verhindern, ist schon lange bekannt. Verwendung finden hier in aller Regel Aluminium- und Aluminium/Zirkoniumsalze. Die verringerte Schweißproduktion hat nach neuesten Erkenntnissen keine Auswirkung auf den Organismus, da die 'Kühlwirkung' zum großen Teil über das 'Schwitzen' der anderen Hautpartien (ekkrine Drüsen) erfolgt.
Die Hemmung des Bakterienwachstums durch Bakteriostatika im Bereich der mit apokrinen Schweißdrüsen durchsetzten Hautzonen ist nicht unbedenklich und führt zum Teil zu starken Reizungen und allergischen Reaktionen.
Als Bakteriozid wirkt auch der Alkohol (Ethanol), der in vielen der herkömmlichen Deoprodukte enthalten ist. Auch hier sind Nebenwirkungen häufig.
Zur Maskierung des Schweißgeruchs sind in der Regel Duftstoffe bzw. Parfümstoffe in den Deozubereitungen vorhanden. Diese wirken zum Teil auch bakteriostatisch, haben aber bei vielen Anwendern ähnliche Nebenwirkungen wie die Bakteriostatika.
Aufgrund der zuvor beschriebenen Wirkungsweise und den damit einhergehenden Nebenwirkungen sind die Bestrebungen groß, Deoprodukte zu entwickeln, die keine Nebenwirkungen aufweisen. Der Trend geht also eindeutig in Richtung Kombinationsprodukt, bei dem eine desodorierende und antitranspirante Wirkung mit hautpflegender Wirkung einhergeht.
Solche Pflegedeos auf Basis von O/W-Emulsionen sind schon im Markt eingeführt, die Handhabung, sprich die Aufbringung auf die Haut, lässt aber noch stark zu wünschen übrig.
Viele Verbraucher favorisieren für die Auftragung von Antitranspirantien die Produktform der sog. Pumpzerstäuber, da sich das Füllgut aus diesen fein verteilt in die Achselregion aufbringen lässt, ohne dass die Finger mit diesem in Berührung gebracht werden müssen. Gegenüber Aerosolen besteht weiterhin der ökologische Vorteil, dass Pumpzerstäuber ohne die Verwendung von Treibmitteln (verflüssigte Gase) auskommen. Vor allem aus ästhetischen Gründen werden transparente und transluzente Produkte von vielen Verbrauchern bevorzugt. Die Kombination dieses Merkmals mit dem Wunsch nach gut wirksamen Antitranspirantprodukten ließ sich bislang nur mit wässrig-alkoholischen Rezepturen realisieren. Diese Formulierungen bestehen praktisch nur aus Wasser und Alkohol als Medium, Deo- und Antitranspirantmittel als Wirkstoffe sowie Parfüm, Löslichkeitsvermittler und Verdicker (zumeist auf Kohlenhydratbasis) als zusätzliche Agenzien. Sie werden vom Verbraucher als frisch und kühlend empfunden, sind aber gleichzeitig mit einer ganzen Reihe an Mankos behaftet. So ist die Applikation vor allem auf frisch rasierter Haut durch den Alkoholgehalt mit Unverträglichkeiten verbunden. Ein weiterer großer Nachteil ist die Tatsache, dass in derartige Systeme keine größeren Ölmengen eingearbeitet werden können. Durch den für eine hoch-effektive Wirkleistung erforderlichen hohen Gehalt an Antitranspirantsalz verbleibt nach der Applikation auf der Haut ein weißer Rückstand, der vom Verbraucher als überaus störend empfunden wird. Durch die technologisch bedingte Abwesenheit einer ausreichendend großen Ölphase kann dieser allerdings nicht kaschiert werden. Darüber hinaus führt die Verwendung von Kohlenhydrat-Verdickern zu einer gewissen Klebrigkeit des Produktes nach dem Verdunsten des Alkohols. Zusammenfassend kann man sagen, daß wässrigalkoholische Rezepturen nicht als Grundlage für die Einarbeitung hoher Gehalte an Antitranspirantwirkern (Aluminium- bzw. Aluminium/Zirkonium-Komplexe) geeignet sind.

Die Lösung all dieser Nachteile ließ lange auf sich warten. Erst in jüngster Zeit sind auch kosmetisch ansprechende alkoholfrei-transparente Produkte möglich, die auf sog. Mikroemulsionen basieren. Diese haben den großen Vorteil, dass man auch größere Mengen an verschiedenen Ölen - mit all den oben beschriebenen positiven Effekten für den Verbraucher - stabil einarbeiten kann. Formulierungen dieser Art sind prinzipiell mittels Phaseninversionstemperatur-Technologie (PIT) oder Hochdruckhomogenisierung zugänglich. Die notwendige Stabilität des Emulgatorsystems gegenüber hohen Konzentrationen an Antitranspirantsalzen stellt jedoch hohe Anforderungen an die Formulierungskunst des Produktentwicklers.
Die Verwendung von Mikroemulsionen im kosmetischen Bereich wird eingehend in EP0814752 beschrieben.

Viele Verbraucher favorisieren für die Auftragung von Antitranspirantien die Produktform der sog. Pumpzerstäuber, da sich das Füllgut aus diesen fein verteilt in die Achselregion aufbringen lässt, ohne dass die Finger mit diesem in Berührung gebracht werden müssen. Gegenüber Aerosolen besteht weiterhin der ökologische Vorteil, dass Pumpzerstäuber ohne die Verwendung von Treibmitteln (verflüssigte Gase) auskommen. Mit herkömmlichen Zerstäuberpumpen ist jedoch keine einheitliche Tröpfchengröße und kein gleichmäßiges Sprühbild erreichbar, da viele Formulierungen mit einem hohen Gehalt an Antitranspirantwirker, vor allem auf einer wässrig-alkoholischen Grundlage basierende, bei seltener Benutzung des Zerstäubers zur Kristallbildung neigen und eine große Verstopfungsgefahr für die Düse darstellen. Damit ist der Produktaustrag be- bzw. verhindert. Andererseits ist die Folge von zu großen Tropfen das Herabfließen der Formulierung (Dripping Effect), wodurch die Anwendung als unangenehm nass und störend empfunden wird.

Aufgabe der vorliegenden Erfindung ist es daher, den Stand der Technik zu bereichern und seinen Nachteilen abzuhelfen.

### Der Zerstäuber

Überraschend hat sich gezeigt, dass die Verwendung von Zerstäuberpumpen mit einem hohem Verhältnis von Vorkompression zur Ausbringungsmenge zu einem kegelförmigen Sprühbild mit gleichmäßig feinen Tröpfchen führt. Damit ist eine gezielte Anwendung in der Achsel gewährleistet und das Zusammenfließen zu großen, abfließenden Tropfen wird verhindert.
Beim Einsatz erfindungsgemäßer Zerstäuber wird durch Betätigen der Zerstäuberpumpe die zu zerstäubende Flüssigkeit in einer zylindrischen Kammer durch Herabdrücken eines Kolbens unter Druck gesetzt und 'vorkomprimiert'. Erreicht die Vorkompression einen Druck von rund 0,7 MPa, öffnet sich ein Pumpenventil und die Flüssigkeit kann in Richtung der Düse strömen. Dabei wird die Flüssigkeit durch zwei oder mehrere strahlenförmig zu einer zylindrischen Düsenöffnung zulaufende Wirbelkanäle gepresst und nach Passage der Düsenöffnung zerstäubt.
Mit Vorkompression ist der Druck gemeint, der aufgebaut werden muss, um das Ventil zur Außenwelt zu öffnen und den Inhalt durch die Düse hindurch zu versprühen. Da es sich um gasfreie Systeme handelt, ist die Kompression nicht mit einer Volumenänderung gleichzusetzen, sondern einer dynamischen Druckerhöhung. Die Wirbelkanäle versetzen die strömende Flüssigkeit in eine Rotationsbewegung um die Fließachse.
Durch den gegenüber normalen Zerstäuberpumpen erhöhten Anfangsdruck, mit dem das Füllgut durch die Austrittsdüse gedrückt wird, und der speziellen Kombination von schwerflüchtigen Zubereitungsbestandteilen, wird die Verstopfung der Düse verhindert. Dadurch ist auch der Einsatz von den im Folgenden beschriebenen Emulsionsformulierungen mit erhöhtem Antitranspirantgehalt möglich, da eventuell entstandene Kristalle, Rückstände und Verkrustungen im Bereich der Düse durch den hohen Sprühdruck abgesprengt werden.

Das Sprühbild bleibt während des gesamten Sprühvorganges - dessen zeitlicher Rahmen durch die Zeitspanne des Herunterdrückens des Kolbens bestimmt wird - gleich, da das oben beschriebene Ventil als eine Art Überdruckventil fungiert und nur bei entsprechendem Vordruck öffnet und so der Sprühdruck sich über den gesamten Zeitraum nicht ändert.
Die erfindungsgemäßen Zerstäuber haben eine sehr geringe Betätigungskraft und weisen im Vergleich zu normalen Zerstäubern geringe Sprühvolumina auf. Die Wirkungsweise des geringen Sprühvolumens ist durch das sehr gleichmäßige und feine Sprühbild dem von herkömmlichen Zerstäubern überlegen.

### Die Formulierung

Erstaunlicherweise lassen sich als Basisformulierung für die oben genannten Aufgaben alkoholfreie transparente oder transluzente Mikroemulsionen vom Typ Öl-in-Wasser,
- umfassend eine Ölphase und eine Wasserphase
- enthaltend:
   einen oder mehrere polyethoxylierte O/W-Emulgatoren und/oder
   einen oder mehrere polypropoxylierte O/W-Emulgatoren und/oder
   einen oder mehrere polyethoxylierte und polypropoxylierte O/W-Emulgatoren,
- gewünschtenfalls ferner enthaltend einen oder mehrere W/O-Emulgatoren
- einen Emulgatorgehalt kleiner als 20 Gew.-%, bezogen auf das Gesamtgewicht der Emulsion,
- einen Antitranspirantgehalt von 5 bis 40 Gew.-%, insbesondere von 7 bis 25 Gew.- %, bezogen auf das Gesamtgewicht der Emulsion, aufweisend,
- erhältlich auf die Weise, dass man ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe auf eine Temperatur innerhalb oder oberhalb des Phaseninversionstemperaturbereiches bringt, und hernach auf Raumtemperatur abkühlt,
wie in EP0814752 beschrieben, hervorragend für Antitranspirantprodukte einsetzen.

Erfindungsgemäße Mikroemulsionen haben eine niedrige Viskosität, sind versprühbar, eignen sich vorzüglich als Vehikel für verschiedenste Wirkstoffe, insbesondere lipidlösliche Wirkstoffe und zeichnen sich darüber hinaus durch vorzügliche Haut- und Schleimhautverträglichkeit aus.

Alle Bestandteile - bis auf Wasser und Duftstoffe - der erfindungsgemäßen Mikroemulsion sind schwerflüchtig, d.h. sie weisen im reinen Zustand einen geringen Dampfdruck bei 25°C auf, wodurch ein Eintrocknen sowie Kristallbildung bei regelmäßiger Verwendung des Zerstäubers unterbunden wird.

Vorteilhaft im Sinne der Erfindung ist es, wenn die Ölphase der Öl-in-Wasser-Emulsion eine Tröpfchengröße kleiner 100 nm aufweist.

Vorteilhaft wird oder werden der polyethoxylierte bzw. polypropoxylierte bzw. polyethoxylierte und polypropoxylierte O/W-Emulgator oder die polyethoxylierten bzw. polypropoxylierten bzw. polyethoxylierten und polypropoxylierten O/W-Emulgatoren gewählt aus der Gruppe
- der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl von 10 bis 80 darstellen
- der Fettsäureethoxylate der allgemeinen Formel R-COO-(-CH₂-CH₂-O-)ₙ -H, wobei R einen verzweigten oder unverzweigten Alkyl- oder Alkenylrest und n eine Zahl von 10 bis 40 darstellen,
- der veretherten Fettsäureethoxylate der allgemeinen Formel R-COO-(-CH₂-CH₂-O-)ₙ -R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweigter Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙ-SO₃-H mit kosmetisch oder pharmazeutisch akzeptablen Kationen, wobei R einen verzweigten oder unverzweigten Alkyl- oder Alkenylrest mit 5-30 C-Atomen und n eine Zahl von 1 bis 50 darstellen.
- der Fettalkoholpropoxylate der allgemeinen Formel R-O-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolether der allgemeinen Formel R-O-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel R-COO-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel R-COO-(-CH₂-CH(CH₃)-O-)ₙC(O)-R', wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel R-COO-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweiger Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100
- der Alkylethercarbonsäuren der allgemeinen Formel R-O-(-CH₂-CH(CH₃)O-)ₙ-CH₂-COOH bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel R-O-(-CH₂-CH(CH₃)-O-)ₙ-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel R-O-Xₙ-Yₘ-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel R-O-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel R-COO-Xₙ-Yₘ-R', wobei R und R' unabhängig voneinander verzweigte oder unverzweigte Alkyl- oder Alkenylreste darstellen, wobei X und Y. nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel R-COO-Xₙ-Yₘ-H, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

Insbesondere ist vorteilhaft, wenn der polyethoxylierte bzw. polypropoxylierte bzw. polyethoxylierte und polypropoxylierte O/W-Emulgator oder die polyethoxylierten bzw. polypropoxylierten bzw. polyethoxylierten und polypropoxylierten O/W-Emulgatoren gewählt wird oder werden aus der Gruppe
- der Fettalkoholethoxylate der allgemeinen Formel R-O-(-CH₂-CH₂-O-)ₙ-H, wobei R einen verzweigten oder unverzweigten Alkyl- oder Alkenylrest mit 5 - 30 C-Atomen und n eine Zahl von 10 bis 25 darstellen
- der ethoxylierten Wollwachsalkohole mit HLB-Werten von 11-16, ganz besonders vorteilhaft mit HLB-Werten von 14,5 - 15,5.
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste mit 5 -30 C-Atomen und n eine Zahl von 10 bis 25 darstellen,
- der Fettsäureethoxylate der allgemeinen Formel R-COO-(-CH₂-CH₂-O-)ₙ -H, wobei R einen verzweigten oder unverzweigten Alkyl- oder Alkenylrest mit 5 - 30 C-Atomen und n eine Zahl von 10 bis 25 darstellen,
- der veretherten Fettsäureethoxylate der allgemeinen Formel R-COO-(-CH₂-CH₂-O-)ₙ-R', wobei R und R' unabhängig voneinander verzweigte oder unverzweigte Alkyl- oder Alkenylreste mit 5 - 30 C-Atomen und n eine Zahl von 10 bis 50 darstellen,
- der veresterten Fettsäureethoxylate der allgemeinen Formel R-COO-(-CH₂-CH₂-O-)ₙ -C(O)-R', wobei R und R' unabhängig voneinander verzweigte oder unverzweigte Alkyl- oder Alkenylreste mit 5 - 30 C-Atomen und n eine Zahl von 10 bis 50 darstellen,
- der Polyethylenglycolglycerinfettsäureester gesättigter und/oder ungesättigter, verzweigter und/oder unverzweigter Fettsäuren mit 6 bis 26 C-Atomen und einem Ethoxylierungsgrad zwischen 3 und 40
- der ethoxylierten Sorbitanester mit einem Ethoxylierungsgrad von 3 bis 30
- der Cholesterinethoxylate mit HLB-Werten von 11 - 16, ganz besonders vorteilhaft mit HLB-Werten von 14,5 - 15,5
- der ethoxylierten Triglyceride mit HLB-Werten von 11 - 16, ganz besonders vorteilhaft mit HLB-Werten von 14,5 -15,5
- der Alkylethercarbonsäuren der allgemeinen Formel R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 10 bis 20 darstellen,
- der Polyoxyethylensorbitolfettsäureester, basierend auf verzweigten oder unverzweigten Alkan- oder Alkensäuren und einen Ethoxylierungsgrad von 10 bis 80 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙ -SO₃-H mit kosmetisch oder pharmazeutisch akzeptablen Kationen, wobei R einen verzweigten oder unverzweigten Alkyl- oder Alkenylrest mit 5-30 C-Atomen und n eine Zahl von 3 bis 30 darstellen,
- der Fettalkoholpropoxylate der allgemeinen Formel R-O-(-CH₂-CH(CH₃)-O-)ₙ-H, wobei R einen verzweigten oder unverzweigten Alkyl- oder Alkenylrest mit 5-30 C-Atomen und n eine Zahl von 10 bis 30 darstellen,
- der Polypropylenglycolether der allgemeinen Formel R-O-(-CH₂-CH(CH₃)-O-)ₙ-R', wobei R und R' unabhängig voneinander verzweigte oder unverzweigte Alkyl- oder Alkenylreste mit 5-30 C-Atomen und n eine Zahl von 10 bis 40 darstellen,
- der propoxylierten Wollwachsalkohole mit HLB-Werten von 11 - 16, ganz besonders vorteilhaft mit HLB-Werten von 14,5 - 15,5,
- der Fettsäurepropoxylate der allgemeinen Formel R-COO-(-CH₂-CH(CH₃)-O-)ₙ-H, wobei R einen verzweigten oder unverzweigten Alkyl- oder Alkenylrest mit 5-30 C-Atomen und n eine Zahl von 10 bis 40 darstellen,
- der veretherten Fettsäurepropoxylate der allgemeinen Formel R-COO-(-CH₂-CH(CH₃)-O-)ₙ-R', wobei R und R' unabhängig voneinander verzweigte oder unverzweigte Alkyl- oder Alkenylreste mit 5-30 C-Atomen und n eine Zahl von 10 bis 30 darstellen,
- der veresterten Fettsäurepropoxylate der allgemeinen Formel R-COO-(-CH₂-CH(CH₃)-O-)ₙ-C(O)-R', wobei R und R' unabhängig voneinander verzweigte oder unverzweigte Alkyl- oder Alkenylreste mit 5-30 C-Atomen und n eine Zahl von 10 bis 50 darstellen,
- der Polypropylenglycolglycerinfettsäureester gesättigter und/oder ungesättigter, verzweigter und/oder unverzweigter Fettsäuren mit 6 bis 26 C-Atomen und einem Propoxylierungsgrad zwischen 3 und 50
- der propoxylierten Sorbitanester mit einem Propoxylierungsgrad von 3 bis 80
- der Cholesterinpropoxylate mit HLB-Werten von 11 - 16, ganz besonders vorteilhaft mit HLB-Werten von 14,5 - 15,5
- der propoxylierten Triglyceride mit HLB-Werten von 11 - 16, ganz besonders vorteilhaft mit HLB-Werten von 14,5 - 15,5
- der Alkylethercarbonsäuren der allgemeinen Formel R-O-(CH₂-CH(CH₃)O)ₙ- CH₂-COOH bzw. deren kosmetisch oder pharmazeutisch akzeptablen Salze, wobei R einen verzweigten oder unverzweigten Alkyl- oder Alkenylrest mit 5-30 C-Atomen und n eine Zahl von 10 bis 30 darstellen,
- der Alkylethersulfate bzw. die diesen Sulfaten zugrundeliegenden Säuren der allgemeinen Formel R-O-(-CH₂-CH(CH₃)-O-)ₙ-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 30 darstellen.

Erfindungsgemäß besonders.vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 16, ganz besonders vorteilhaft mit HLB-Werten von 14,5 - 15,5, sofern die O/W-Emulgatoren gesättigte Reste R und R' aufweisen. Weisen die O/W-Emulgätoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol-(18)stearylether (Steareth-18), Polyethylenglycol(19)stearylether (Steareth-19), Polyethylenglycol(20)stearylether (Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol-(18)isostearylether (Isosteareth-18), Polyethylenglycol(19)isostearylether (Isosteareth-19-), Polyethylenglycol(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (lsoceteth-17), Polyethylenglycol(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).
Polyethylenglycol(13)cetylstearylether (Ceteareth-13), Polyethylenglycol(14)cetylstearyl-ether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20),

Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol-(14)isostearat, Polyethylenglycol(15)isostearat, Polyethylenglycol(16)isostearat, Polyethylenglycol(17)isostearat, Polyethylenglycol(18)isostearat, Polyethylenglycol(19)isostearat, Polyethylenglycol(20)isostearat, Polyethylenglycol(21)isostearat, Polyethylenglycol-(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natriumlaureth-14-sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)-sorbitanmonooleat zu wählen.

Als fakultative, dennoch erfindungsgemäß vorteilhafte W/O-Emulgatoren können eingesetzt werden: Fettalkohole mit 8 bis 30 Kohlenstoffatomen; Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Es ist erfindungsgemäß möglich, den Gesamtgehalt an Emulgatoren kleiner als 20 Gew.- %, bezogen auf das Gesamtgewicht der Mikroemulsion, zu halten. Es wird bevorzugt, den Gesamtgehalt an Emulgatoren kleiner als 15 Gew.-%, insbesondere kleiner als 10 Gew.-%, bezogen auf das Gesamtgewicht der Mikroemulsion, zu halten.

Die Ölphase der erfindungsgemäßen Mikroemulsionen wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen.

Es kann auch gegebenenfalls vorteilhaft sein, Wachse, beispielsweise Cetylpalmitat, als alleinige Lipidkomponente der Ölphase einzusetzen. In solchen Fällen können die erfindungsgemäßen O/W-Mikroemulsionen auch gegebenenfalls als Mikrodispersionen fester Wachspartikel anfallen.

Vorteilhaft wird die Ölphase gewählt aus der Gruppe 2-Ethylhexylisostearat, Octyldodecanol, lsotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Octamethylcyclotetrasiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Herstellung der erfindungsgemäßen Mikroemulsionen erfolgt vorteilhaft dergestalt, dass man ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe, welche unterhalb des Phaseninversionstemperaturbereiches eine O/W-Emulsion bilden, auf eine Temperatur oberhalb oder innerhalb des Phaseninversionstemperaturbereiches bringt, und die gebildete Mikroemulsion hernach auf Raumtemperatur abkühlt. Dies geschieht bevorzugt unter Rühren.

Erstaunlicherweise ist es jeweils möglich, auf einen Homogenisierungsschritt zu verzichten.

Vorteilhaft lassen sich große Mengen saurer Aluminium- und/oder Aluminium/Zirkoniumsalze stabil in die Emulsionen einarbeiten. Es können 5 bis 40 Gew.-%, insbesondere 7 bis 25 Gew.-% Aluminiumchlorhydrat und/oder Aluminium/Zirkoniumchlorhydrat stabil in die Emulsionen eingearbeitet werden. Hierbei beziehen sich die beschriebenen Konzentrationsbereiche auf die sog. Aktivgehalte der Antitranspirant-Komplexe: bei den Aluminium-Verbindungen auf wasserfreie Komplexe, bei den Aluminium/Zirkonium-Verbindungen auf wasser- und pufferfreie Komplexe. Als Puffer wird hier üblicherweise Glycin verwendet.

Die nachfolgende Auflistung vorteilhaft einzusetzender Antitranspirant-Wirker soll in keinster Weise einschränkend sein:

Aluminium-Salze (der empirischen Summenformel [Al₂(OH)ₘClₙ], wobei m+n=6):
- Aluminium-Salze wie Aluminiumchlorid AlCl₃, Aluminiumsulfat Al₂(SO₄)₃
- Aluminiumchlorhydrat [Al₂(OH)₅Cl] x H₂O Standard Al-Komplexe: Locron L (Clariant), Chlorhydrol (Reheis), ACH-303 (Summit), Aloxicoll L (Giulini). Aktivierte Al-Komplexe: Reach 501 (Reheis), AACH-324 (Summit)
- Aluminiumsesquichlorhydrat [Al₂(OH)_{4,5}Cl_{1,5}] x H₂O Standard Al-Komplexe: Aluminum Sesquichlorohydrate (Reheis), ACH-308 (Summit), Aloxicoll 31 L (Giulini) Aktivierte Al-Komplexe: Reach 301 (Reheis)
- Aluminiumdichlorhydrat [Al₂(OH)₄Cl₂] x H₂O

Aluminium-Zirkonium-Salze:
- Aluminium/Zirkonium Trichlorhydrex Glycin [Al₄Zr(OH)₁₃Cl₃] x H₂O x Gly Standard Al/Zr-Komplexe: Rezal 33GP (Reheis), AZG-7164 (Summit), Zirkonal P3G (Giulini) Aktivierte Al/Zr-Komplexe: Reach AZZ 902 (Reheis), AAZG-7160 (Summit), Zirkonal AP3G (Giulini)
- Aluminium/Zirkonium Tetrachlorhydrex Glycin [Al₄Zr(OH)₁₂Cl₄] x H₂O x Gly Standard Al/Zr-Komplexe: Rezal 36G (Reheis), AZG-368 (Summit), Zirkonal L435G (Giulini) Aktivierte Al/Zr-Komplexe: Reach AZP 855 (Reheis), AAZG-6313-15 (Summit), Zirkonal AP4G (Giulini)
- Aluminium/Zirkonium Pentachlorhydrex Glycin [Al₈Zr(OH)₂₃Cl₅] x H₂O x Gly Standard Al/Zr-Komplexe: Rezal 67 (Reheis), Zirkonal L540 (Giulini) Aktivierte Al/Zr-Komplexe: Reach AZN 885 (Reheis)
- Aluminium/Zirkonium Octachlorhydrex Glycin [Al₈Zr(OH)₂₀Cl₈] x H₂O x Gly

Ebenso von Vorteil können aber auch Glycin-freie Aluminium/Zirkonium-Salze sein.

Dabei soll die Verwendung der Antitranspirant-Wirker aus den Rohstoffklassen Aluminium- und Aluminium/Zirkonium-Salzen nicht auf die handelsüblichen zumeist wässrigen Lösungen, wie z.B. Locron L (Clariant), beschränkt sein, sondern es kann auch von Vorteil sein, die ebenfalls handelsüblichen wasserfreien Pulver derselbigen Rohstoffe durch Einbringung in die beanspruchten Formulierungen zum Einsatz zu bringen, wie z.B. Locron P (Clariant).

Vorteilhaft könnte auch die Verwendung von sog. AT-Salz Suspensionen sein, bei denen pulverförmig vorliegende Aluminium- und Aluminium/Zirkonium-Salze in diversen Ölen dispergiert angeboten werden.

Des weiteren kann es aber auch von Vorteil sein, spezielle Aluminium- und Aluminium/Zirkonium-Salze zum Einsatz zu bringen, die zur Löslichkeitsverbesserung als Glykol-Komplexe angeboten werden.

Weitere vorteilhafte Antitranspirant-Wirker basieren anstelle von Aluminium bzw. Zirkonium auf anderen Metallen, wie z.B. Beryllium, Titan, Hafnium.

Dabei soll die Liste der verwendbaren Antitranspirant-Wirker aber nicht auf metallhaltige Rohstoffe begrenzt sein, sondern von Vorteil sind auch Verbindungen, die Nichtmetalle wie Bor enthalten sowie solche, die dem Bereich der organischen Chemie zuzurechnen sind, wie z.B. Anticholinergika.
Vorteilhaft sind in diesem Sinne auch Polymere, die sowohl metallhaltig als auch metalfrei sein können.

Der in zahlreichen Zubereitungen auftretende Effekt, dass nach dem Auftragen der Zubereitung auf der Haut ein sichtbarer weißlicher Rückstand zurückbleibt, wird in der Regel vom Anwender als störend empfunden. In wasserfreien Zubereitungen hat sich der Einsatz von propoxylierten Alkoholen zur Kaschierung dieser Erscheinung bewährt. Im Falle von wasserhaltigen Zubereitungen ist bisher keine zufriedenstellende Lösung dieses Problems bekannt. Der Zusatz von propoxylierten Alkoholen mit 10 bis 20 Propyloxyeinheiten und 2 bis 10 Kohlenstoffatomen in der Alkylkette, insbesondere PPG-14-Butylether, als Bestandteil der mittelpolaren Ölphase hilft dem beschriebenen Mangel des Standes der Technik ab, indem das Auftreten derartiger weißlicher Rückstände zuverlässig kaschiert wird.

Vorteilhaft können erfindungsgemäßen Zubereitungen Desodorantien zugesetzt werden. Den üblichen kosmetischen Desodorantien liegen unterschiedliche Wirkprinzipien zugrunde.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt. Auch die Kombination von Adstringentien mit antimikrobiell wirksamen Stoffen in ein und derselben Zusammensetzung ist gebräuchlich.

Alle für Desodorantien gängigen Wirkstoffe können vorteilhaft genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre. Ammoniumverbindungen, Nelkenöl, Minzöl, Thymianöl, Triethylcitrat, Farnesol (3,7,11-Trimethyl-2,6,10-dodecatriën-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden.

Die Liste der genannten Wirkstoffe bzw. Wirkstoffkombinationen, die in den erfindungsgemäßen Emulsionen verwendet werden können, soll selbstverständlich nicht limitierend sein.
Die Menge der Desodorantien (eine oder mehrere Verbindungen) in den Zubereitungen beträgt vorzugsweise 0,01 bis 10 Gew.-%, besonders bevorzugt 0,05 bis 5 Gew.-%, insbesondere 0,1 bis 1 Gew.-%, bezogen auf das Gesamtgewicht der Zubereitung.

Erfindungsgemäße Zubereitungen können auch zusätzlich Hydrokolloide, anorganische Pigmente, Antioxidantien und/oder kosmetische oder dermatologische Wirkstoffe enthalten, die sowohl öl- als auch wasserlöslich sein können.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, organische Lösungsmittel oder Silikonderivate sowie Moisturizer.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispielrezepturen

**transluzente Mikroemulsions-Formeln:**

| **Chemische Bezeichnung** | **INCI** | **Nr. 1** | **Nr. 2** | **Nr. 3** | **Nr. 4** |
|---|---|---|---|---|---|
| Polyoxyethylen(20)cetylstearylether | Ceteareth-20 | 3 | 4 | 5 | 4 |
| Polyoxyethylen(12)cetylstearylether | Ceteareth-12 | 0,5 | - | - | - |
| Glycerinstearat | Glyceryl Stearate | 3 | 3 | 2 | - |
| Glycerinisostearat | Glyceryl Isostearate | - | - | - | 3 |
| Cetylstearylalkohol | Cetearyl Alcohol | 0,5 | - | - | - |
| Cetylpalmitat | Cetyl Palmitate | 0,5 | - | - | - |
| Cetylalkohol | Cetyl Alcohol | - | 2 | - | 1 |
| Stearylalkohol | Stearyl Alcohol | - | - | 2 | - |
| Capryl-Caprinsäureester | Coco-Caprylate/Caprate | 5 | 3 | 3 | 4 |
| Di-n-Octylether | Dicaprylyl Ether | 5 | - | 5 | 4 |
| Di-n-Octylcarbonat | Dicaprylyl Carbonate | - | 3 | - | 1 |
| Glycerin | Glycerin | 4 | 2 | 3 | 3 |
| Aluminium Chlorohydrat (50% wäßr. Lös.) | Aluminum Chlorohydrate | 16 | 30 | 40 | 20 |
| 3.7.11-Trimethyl-2.6.10-dodecatrien-1-ol | Farnesol | 1 | - | - | 1 |
| Octyldodecanol | Octyldodecanol | - | 1 | 1 | - |
| Avocadoöl | Persea Gratissima | 1 | 1 | 1 | - |
| Glycerinlaurat | Glyceryl Laurate | 1 | - | 1 | - |
| Parfüm | Parfum | 1 | 1 | - | - |
| Wasser, ad | Aqua, ad | 100 | 100 | 100 | 100 |

**transparente Mikroemulsions-Formeln:**

| **Chemische Bezeichnung** | **INCI** | **Nr. 5** | **Nr. 6** | **Nr. 7** | **Nr. 8** |
|---|---|---|---|---|---|
| Glycerinmonoisostearat | GlycerylIsostearate | 3 | 2 | 3 | 4 |
| Polyoxyethylen-20-isohexadecylether | Isoceteth-20 | 6 | 5 | - | - |
| Polyoxyethylen-20-isooctadecylether | Isosteareth-20 | - | - | 6 | - |
| Polyoxyethylen-25-octyldodecylether | Octyldodeceth-25 | - | - | - | 5 |
| Capryl-Caprinsäureester | Coco-Caprylate/Caprate | - | 5 | 3 | 5 |
| Di-n-Octylether | Dicaprylyl Ether | 5 | - | - | - |
| Di-n-Octylcarbonat | Dicaprylyl Carbonate | - | 3 | 5 | 3 |
| Glycerin | Glycerin | - | 4 | 3 | 3 |
| Butylenglykol | Butylene Glycol | 3 | - | - | - |
| Aluminium Chlorohydrat (50% wäßr. Lös.) | Aluminum Chlorohydrate | 16 | 20 | 40 | 30 |
| 3.7.11-Trimethyl-2.6.10-dodecatrien-1-ol | Farnesol | 1 | - | 1 | - |
| Avocadoöl | Persea Gratissima | - | 1 | - | 1 |
| Octyldodecanol | Octyldodecanol | - | 1 | - | 1 |
| Glycerinlaurat | Glyceryl Laurate | - | 1 | - | 1 |
| Glycerinmonocaprat | Glyceryl Caprate | 1 | - | 1 | - |
| Jojobaöl | Buxus Chinensis | 1. | - | 1 | - |
| Parfüm | Parfum | 1 | 1 | - | 1 |
| Wasser, ad | Aqua, ad | 100 | 100 | 100 | 100 |

## Patentansprüche

1. Antitranspirantprodukt, enthaltend alkoholfreie transparente oder transluzente Mikroemulsionen vom Typ Öl-in-Wasser (O/W),
- umfassend eine Ölphase und eine Wasserphase,
- enthaltend:
einen oder mehrere polyethoxylierte O/W-Emulgatoren und/oder
einen oder mehrere polypropoxylierte O/W-Emulgatoren und/oder
einen oder mehrere polyethoxylierte und polypropoxylierte O/W-Emulgatoren,
- gewünschtenfalls ferner enthaltend einen oder mehrere W/O-Emulgatoren
- einen Emulgatorgehalt kleiner als 20 Gew.-%, bezogen auf das Gesamtgewicht der Emulsion,
- einen Antitranspirantgehalt von 5 bis 40 Gew.-%, insbesondere von 7 bis 25 Gew.-%, bezogen auf das Gesamtgewicht der Emulsion, aufweisend,
- erhältlich auf die Weise, dass man ein Gemisch aus den Grundkomponenten, umfassend Wasserphase, Ölphase, einen oder mehrere der erfindungsgemäßen O/W-Emulgatoren, gewünschtenfalls einen oder mehrere W/O-Emulgatoren, sowie gewünschtenfalls weitere Hilfs-, Zusatz- und/oder Wirkstoffe auf eine Temperatur innerhalb oder oberhalb des Phaseninversionstemperaturbereiches bringt, und hernach auf Raumtemperatur abkühlt,
einen Pumpsprüher, bestehend aus
- einem Behältnis und
- einer Zerstäuberpumpe folgende Merkmale aufweisend:
- ein Steigrohr,
- zylindrische Kammer, die durch Herabdrücken eines Kolbens unter Druck gesetzt wird,
- einem Pumpenventil, das die zylindrische Kammer verschließt und bei einem Druck von mindestens 0,7 MPa öffnet,
- zwei oder mehr strahlenförmig zu einer Düsenöffnung zulaufende Wirbelkanäle, die die strömende Flüssigkeit in eine Rotationsbewegung zur Fließachse versetzen,
zur Verhinderung der übermäßigen Geruchs- und Schweißabsonderung der Haut.

2. Antiranspirantprodukt nach Anspruch 1, **dadurch gekennzeichnet, dass** die Antitranspirant-Wirkstoffe saure Salze sind.

3. Antiranspirantprodukt nach einem oder mehreren der vorhergehenden Ansprüche, **dadurch gekennzeichnet, dass** die Ölphase der Öl-in-Wasser-Emulsion eine Tröpfchengröße kleiner 100 nm aufweist.

4. Antiranspirantprodukt nach einem oder mehreren der vorhergehenden Ansprüche, **dadurch gekennzeichnet, dass** die sauren Salze eine oder mehrere saure Aluminium- und/oder Aluminium/Zirkoniumsalze sind und die Gesamtmenge an dem oder den sauren Aluminium- und/oder Aluminium/Zirkoniumsalzen mindestens 5 Gew.-% bezogen auf das Gesamtgewicht der Zubereitungen beträgt.

5. Antiranspirantprodukt nach einem oder mehreren der vorhergehenden Ansprüche, **dadurch gekennzeichnet, dass** die Gesamtmenge an einem oder mehreren polyethoxylierten O/W-Emulgatoren in den fertigen Zubereitungen aus dem Bereich von 0,1 bis 8 Gew.-%, bevorzugt von 0,5 bis 6,5 Gew.-%, besonders bevorzugt von 1 bis 5 Gew.-% gewählt wird, bezogen auf das Gesamtgewicht der Zubereitungen.

6. Antiranspirantprodukt nach einem oder mehreren der vorhergehenden Ansprüche, **dadurch gekennzeichnet, dass** die Gesamtmenge an einem oder mehreren polypropoxylierten O/W-Emulgatoren in den fertigen Zubereitungen aus dem Bereich von 0,1 bis 8 Gew.-%, bevorzugt von 0,5 bis 6,5 Gew.-%, besonders bevorzugt von 1 bis 5 Gew.-% gewählt wird, bezogen auf das Gesamtgewicht der Zubereitungen.

7. Antiranspirantprodukt nach einem oder mehreren der vorhergehenden Ansprüche, **dadurch gekennzeichnet, dass** die Gesamtmenge an einem oder mehreren polyethoxylierten und polypropoxylierten O/W-Emulgatoren in den fertigen Zubereitungen aus dem Bereich von 0,1 bis 8 Gew.-%, bevorzugt von 0,5 bis 6,5 Gew.-%, besonders bevorzugt von 1 bis 5 Gew.-% gewählt wird, bezogen auf das Gesamtgewicht der Zubereitungen.

8. Antiranspirantprodukt nach einem oder mehreren der vorhergehenden Ansprüche, **dadurch gekennzeichnet, dass** die Gesamtmenge an einem oder mehreren W/O-Emulgatoren in den fertigen Zubereitungen aus dem Bereich von 0,1 bis 5 Gew.-%, bevorzugt von 0,5 bis 3,5 Gew.-%, besonders bevorzugt von 1 bis 2,5 Gew.-% gewählt wird, bezogen auf das Gesamtgewicht der Zubereitungen.

## Claims

1. Anti-perspirant product comprising alcohol-free transparent or translucent microemulsions of the oil-in-water (O/W) type,
- comprising an oil phase and a water phase,
- comprising:
one or more polyethoxylated O/W emulsifiers and/or
one or more polypropoxylated O/W emulsifiers and/or
one or more polyethoxylated and polypropoxylated O/W emulsifiers,
if desired also comprising one or more W/O emulsifiers
- having an emulsifier content of less than 20% by weight, based on the total weight of the emulsion,
- having an anti-perspirant content of from 5 to 40% by weight, in particular from 7 to 25% by weight, based on the total weight of the emulsion,
- obtainable by bringing a mixture of the basic components comprising water phase, oil phase, one or more of the O/W emulsifiers according to the invention, if desired one or more W/O emulsifiers, and if desired, further auxiliaries, additives and/or active ingredients to a temperature within or above the phase-inversion temperature range, and subsequently cooling it to room temperature,
a pump atomizer consisting of
- a container and
- an atomizer pump having the following features:
- a riser tube,
- cylindrical chamber which is placed under pressure by depressing a piston,
- a pump valve which closes the cylindrical chamber and opens under a pressure of at least 0.7 mPa
- two or more turbulence channels radiating to a nozzle opening, said channels causing the flowing liquid to rotate relative to the flow axis,
for preventing excessive odor and perspiration secretion of the skin.

2. Anti-perspirant product according to Claim 1, **characterized in that** the anti-perspirant active ingredients are acidic salts.

3. Anti-perspirant product according to one or more of the preceding claims, **characterized in that** the oil phase of the oil-in-water emulsion has a droplet size of less than 100 nm.

4. Anti-perspirant product according to one or more of the preceding claims, **characterized in that** the acidic salts are one or more acidic aluminum and/or aluminum/zirconium salts and the total amount of the acidic aluminum and/or aluminum/zirconium salt(s) is at least 5% by weight, based on the total weight of the preparations.

5. Anti-perspirant product according to one or more of the preceding claims, **characterized in that** the total amount of one or more polyethoxylated O/W emulsifiers in the finished preparations is chosen from the range from 0.1 to 8% by weight, preferably from 0.5 to 6.5% by weight, particularly preferably from 1 to 5% by weight, based on the total weight of the preparations.

6. Anti-perspirant product according to one or more of the preceding claims, **characterized in that** the total amount of one or more polypropoxylated O/W emulsifiers in the finished preparations is chosen from the range from 0.1 to 8% by weight, preferably from 0.5 to 6.5% by weight, particularly preferably from 1 to 5% by weight, based on the total weight of the preparations.

7. Anti-perspirant product according to one or more of the preceding claims, **characterized in that** the total amount of one or more polyethoxylated and polypropoxylated O/W emulsifiers in the finished preparations is chosen from the range from 0.1 to 8% by weight, preferably from 0.5 to 6.5% by weight, particularly preferably from 1 to 5% by weight, based on the total weight of the preparations.

8. Anti-perspirant product according to one or more of the preceding claims, **characterized in that** the total amount of one or more W/O emulsifiers in the finished preparations is chosen from the range from 0.1 to 5% by weight, preferably from 0.5 to 3.5% by weight, particularly preferably from 1 to 2.5% by weight, based on the total weight of the preparations.

## Revendications

1. Produit antisudoral, contenant des microémulsions du type huile-dans-eau (H/E) sans alcool, transparentes ou translucides,
- comprenant une phase huileuse et une phase aqueuse,
- contenant:
un ou plusieurs émulsifiants H/E polyéthoxylés et/ou
un ou plusieurs émulsifiants H/E polypropoxylés et/ou
un ou plusieurs émulsifiants H/E polyéthoxylés et polypropoxylés,
- éventuellement contenant en outre un ou plusieurs émulsifiants E/H
- une teneur en émulsifiants inférieure à 20 % en poids, par rapport au poids total de l'émulsion,
- une teneur en antisudoraux de 5 à 40 % en poids, en particulier de 7 à 25 % en poids, par rapport au poids total de l'émulsion,
- pouvant être obtenu en portant à une température dans ou au-dessus de la plage de température d'inversion de phase un mélange des composants de base comprenant phase aqueuse, phase huileuse, un ou plusieurs des émulsifiants H/E selon l'invention, si on le désire un ou plusieurs émulsifiants E/H, ainsi qu'éventuellement d'autres adjuvants, additifs et/ou substances actives, et ensuite en le refroidissant jusqu'à la température ambiante,
- un pulvérisateur à pompe, consistant en
- un récipient et
- une pompe d'atomiseur présentant les caractéristiques suivantes :
- un tube ascendant,
- une chambre cylindrique qui est mise sous pression par poussée par le haut d'un piston,
- une valve de pompe qui ferme la chambre cylindrique et l'ouvre sous une pression d'au moins 0,7 MPa,
- deux ou plus de deux canaux tourbillonnaires radiaux conduisant à un orifice en forme de buse, qui mettent le liquide qui s'écoule en un mouvement de rotation par rapport à l'axe d'écoulement,
pour empêcher la transpiration excessive de la peau et l'émission excessive d'odeur par la peau.

2. Produit antisudoral selon la revendication 1, **caractérisé en ce que** les substances actives antisudorales sont des sels acides.

3. Produit antisudoral selon une ou plusieurs des revendications précédentes, **caractérisé en ce que** la phase huileuse de l'émulsion huile-dans-eau a une taille de gouttelette inférieure à 100 nm.

4. Produit antisudoral selon une ou plusieurs des revendications précédentes, **caractérisé en ce que** les sels acides sont un ou plusieurs sels acides d'aluminium et/ou aluminium/zirconium et la quantité totale du ou des sels acides d'aluminium et/ou aluminium/zirconium est d'au moins 5 % en poids par rapport au poids total des préparations.

5. Produit antisudoral selon une ou plusieurs des revendications précédentes, **caractérisé en ce que** la quantité totale d'un ou plusieurs émulsifiants H/E polyéthoxylés dans les préparations finales est choisie dans la plage allant de 0,1 à 8 % en poids, de préférence de 0,5 à 6,5 % en poids, de façon particulièrement préférée de 1 à 5 % en poids, par rapport au poids total des préparations.

6. Produit antisudoral selon une ou plusieurs des revendications précédentes, **caractérisé en ce que** la quantité totale d'un ou plusieurs émulsifiants H/E polypropoxylés dans les préparations finales est choisie dans la plage allant de 0,1 à 8 % en poids, de préférence de 0,5 à 6,5 % en poids, de façon particulièrement préférée de 1 à 5 % en poids, par rapport au poids total des préparations.

7. Produit antisudoral selon une ou plusieurs des revendications précédentes, **caractérisé en ce que** la quantité totale d'un ou plusieurs émulsifiants H/E polyéthoxylés et polypropoxylés dans les préparations finales est choisie dans la plage allant de 0,1 à 8 % en poids, de préférence de 0,5 à 6,5 % en poids, de façon particulièrement préférée de 1 à 5 % en poids, par rapport au poids total des préparations.

8. Produit antisudoral selon une ou plusieurs des revendications précédentes, **caractérisé en ce que** la quantité totale d'un ou plusieurs émulsifiants E/H dans les préparations finales est choisie dans la plage allant de 0,1 à 5 % en poids, de préférence de 0,5 à 3,5 % en poids, de façon particulièrement préférée de 1 à 2,5 % en poids, par rapport au poids total des préparations.
